# EUROPEAN PATENT SPECIFICATION

(11) **EP 3 741 370 B1**
(45) Date of publication and mention of the grant of the patent: **25.12.2024**
(21) Application number: 19020318.2
(22) Date of filing: 30.04.2019
(51) Int. Cl.: A61K 31/4704, A61P 25/16

(54) **REBAMIPIDE FOR USE IN PREVENTION OF SYNUCLEINOPATHIES**
REBAMIPID ZUR VERWENDUNG BEI DER VORBEUGUNG VON SYNUKLEINOPATHIEN
REBAMIPIDE DESTINÉ À ÊTRE UTILISÉ DANS LA PRÉVENTION DE SYNUCLÉINOPATHIES

(43) Date of publication of application: 25.11.2020
(73) Proprietor: SQUARE POWER LTD, London EC2A 3DQ (GB)
(72) Inventor:

(56) References cited:
- MISHRA AKANKSHA ET AL: "Rebamipide Mitigates Impairments in Mitochondrial Function and Bioenergetics with [alpha]-Synuclein Pathology in 6-OHDA-Induced Hemiparkinson's Model in Rats", NEUROTOXICITY RESEARCH, HARWOOD ACADEMIC PUBLISHERS, LAUSANNE, CH, vol. 35, no. 3, 4 January 2019 (2019-01-04), pages 542 - 562, XP036728349, ISSN: 1029-8428, [retrieved on 20190104], DOI: 10.1007/S12640-018-9983-2
- PEREZ-PARDO PAULA ET AL: "The gut-brain axis in Parkinson's disease: Possibilities for food-based therapies", EUROPEAN JOURNAL OF PHARMACOLOGY, vol. 817, 23 May 2017 (2017-05-23), pages 86 - 95, XP085268480, ISSN: 0014-2999, DOI: 10.1016/J.EJPHAR.2017.05.042
- VAN KAMPEN JACKALINA M. ET AL: "The BSSG rat model of Parkinson's disease: progressing towards a valid, predictive model of disease", THE EPMA JOURNAL, vol. 8, no. 3, 1 September 2017 (2017-09-01), NL, pages 261 - 271, XP093071277, ISSN: 1878-5077, Retrieved from the Internet <URL:http://link.springer.com/content/pdf/10.1007/s13167-017-0114-6.pdf> DOI: 10.1007/s13167-017-0114-6

## Description

### Field of the Invention

The present invention relates to rebamipide for use in a method of preventive treatment of a synucleinopathy.

### Background Art

Synucleinopathies are neurodegenerative disorders associated with the presence of Lewy bodies formed by alpha-synuclein aggregates in brain neurons. It is known that oligomers and aggregates of alpha-synuclein contribute to the death of neurons, whereas the dopamine-dependent neurons are predominantly affected by this mechanism. However, there are further mechanisms of neuronal death which are implied in synucleinopathies, such as Parkinson's disease, including oxidative stress, protein aggregation, reduced mitochondrial activity, proteasomal and lysosomal system dysfunctions.

Alpha-synuclein is a protein of unknown function primarily found in neural tissue. It is found in brain neurons as well as in enteric neural system. Alpha-synuclein is an intrinsically disordered protein; it is assumed that it contains a mix of unstructured alpha-helix and beta-sheet-rich conformers in equilibrium. The aggregation of alpha-synuclein and formation of Lewy bodies is related to increase in the beta conformer proportion (Kingwell K. (2017) Nat Rev Drug Discov; 16(6):371-373). It was suggested that the aggregated alpha-synuclein formed in enteric neural system may enter the brain via vagus nerve and participate in or cause the formation of Lewy bodies (Holmqvist S et al. (2014) Acta Neuropathol; 128(6):805-820).

It was discovered that one of the mechanisms of the misfolding of alpha-synuclein which triggers the aggregate formation might be associated with tiny intestinal wall defects which are sometimes referred to as increased intestinal permeability, including subclinical chronical inflammation (low grade inflammation) of the gut wall (Perez-Pardo P. et al. (2017) Curr Behav Neurosci Rep; 4(4):361-368, Perez-Pardo P. et al. (2017) Eur J Pharmacol; 817:86-95). These findings are further supported by the fact that a proportion of Parkinson's disease patients suffer for many years before the onset of the disease from chronic constipation and gastroparesis which are typical manifestations of little intestinal wall defects - increased intestinal permeability. However, a number of other mechanisms causing misfolding of alpha-synuclein exist, including for example mutations of the gene encoding alpha-synuclein or a lack of chaperones preventing the pathological misfolding.

The current treatment of Parkinson's disease, the most common synucleinopathy, focuses on dopamine supplementation (dopamine precursor levodopa), dopamine agonists, and MAO-B inhibitors preventing dopamine metabolism. It is estimated that about 10 million people worldwide are living with Parkinson's Disease. Providing further options for treatment, or even better, for prevention of Parkinson's disease and further alpha-synucleinopathies, is desirable, in order to delay or prevent the onset of the disease, and in order to address the relevant patient groups.

Rebamipide, which is chemically 2-[(4-chlorobenzoyl)amino]-3-(2-oxo-1H-quinolin-4-yl)propanoic acid, is used for the treatment of gastritis and gastroduodenal ulcers. Its mechanism of action relates to mucosal defense, scavenging free radicals, and temporarily activating genes encoding cyclooxygenase-2. Recently, it has been described to mitigate impairments in mitochondrial function and bioenergetics with alpha-synuclein pathology in 6-OHDA-induced hemiparkinson's model in rats (Mishra A, Krishnamurthy S. (2019) Neurotox Res; 35(3):542-562).

### Disclosure of the Invention

In the framework of the present invention, the inventors have found that rebamipide is capable of preventing and/or reducing the progress of synucleinopathies. The inventors have observed on an animal model of Parkinson's disease that the active ingredient is able to prevent alpha-synuclein aggregation and thus prevents development of synucleinopathies. The presumed mechanism of action is likely based on the ability of rebamipide to induce mucin production in the intestine, to suppress inflammation and to restore the function of the tight junctions of epithelial cells. This leads to recovery of the intestine and its proper function, resulting in reduced permeability of the intestinal wall, which in turn does not allow undesirable foreign substances to pass in the body proper, where they would trigger processes leading to misfolding of alpha-synuclein and its aggregation in the enteric neural system. Since transfer of these aggregates to the brain via vagus nerve leads to emergence of the Lewy bodies and onset of the Parkinson's disease, preventing their formation by normalizing the intestinal permeability constitutes a promising strategy in the treatment of alpha-synucleinopathies, stopping them at the start.

The present invention thus provides rebamipide for use in a method of preventive treatment of a synucleinopathy in persons suffering from increased intestinal permeability or in persons who are at risk of increased intestinal permeability.

Synucleinopathies are disorders caused by alpha-synuclein aggregates forming Lewy bodies. In particular, synucleinopathies include Parkinson's disease, dementia with Lewy bodies and multiple system atrophy. In a preferred embodiment, the synucleinopathy is Parkinson's disease.

"Prevention" or "preventive treatment" shall be understood herein as preventing or delaying the onset of the disease. It is also intended to include delaying the progression of the disease. In such a case rebamipide is administered to a patient who is in an initial or early stage of the disease to be treated in order to slow down its progression.

The present invention provides rebamipide for use in a method of preventive treatment of a synucleinopathy in persons suffering from increased intestinal permeability or in persons who are at risk of increased intestinal permeability, e.g., due to family anamnesis or due to exposure to conditions or substances inducing increased intestinal permeability. It is particularly preferred that rebamipide is for use in a method of prevention of the Parkinson's disease, in persons suffering from increased intestinal permeability or in persons who are at risk of increased intestinal permeability.

In an aspect of the invention, rebamipide is provided for use in a method of progression-reducing treatment of synucleinopathies in persons being in initial or early stage of a synucleinopathy and suffering from increased intestinal permeability or in persons who are at risk of increased intestinal permeability. Progression-reducing treatment should be understood as delaying, reducing or stopping the progression of the synucleinopathy.

"Increased intestinal permeability" is used herein as a term designating little intestinal wall defects, including those caused by subclinical chronical inflammation (low grade inflammation) of the gut wall. These intestinal wall defects may be manifested e.g. by chronic constipation or gastroparesis. Increased intestinal permeability may be diagnosed using specific tests, such as lactulose-mannitol test (LAMA test; e.g., Sequeira I.R. et al. (2014) PLoS One; 9(6):e99256), A-1-AT test, or zonulin test. Typically, increased intestinal permeability is permeability of the intestinal wall to particles having the size of more than 4 Angstroms in radius.

Substances inducing increased intestinal permeability include non-steroidal anti-inflammatory drugs (NSAIDs), such as acetylsalicylic acid, ibuprofen, naproxen, ketoprofen, fenoprofen, flurbiprofen, diclofenac, ketorolac, etodolac, indomethacin, tolmetin, piroxicam, meloxicam and selective COX-2 inhibitors such as celecoxib and etoricoxib; alcohol; nicotine; food additives; antibiotics; and chemotherapeutics. Thus, simultaneous or sequential co-administration of rebamipide with non-steroidal anti-inflammatory drugs, chemotherapeutics or antibiotics may prevent or delay the onset of synucleinopathies caused by the administration of these medicaments. Prophylactic use of rebamipide may also be useful in persons abusing alcohol, nicotine or other drugs. The term "abuse" as used herein is meant to include any consumption, which is not necessary for medical reasons and leads to dependency and/or health impairments including low grade inflammation of the gut wall.

Conditions inducing increased intestinal permeability are related to stress, imbalanced diet, bacterial, viral or parasitic infections and various medical treatments. Such conditions in particular include stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia (e.g. induced by antibiotic treatment).

The persons suffering from increased intestinal permeability typically suffer from at least one condition selected from low grade inflammation of the gut wall, chronic constipation or gastroparesis.

In the therapeutic indications as described in the present invention, rebamipide may preferably be used in oral pharmaceutical forms such as tablets, capsules, dragees, granules, microgranules (sachets), orodispersible tablets or films, sublingual tablets, crushed tablets, oral solutions, oral suspensions, syrups, mouthwashes, rinses; or in rectal pharmaceutical forms such as suppositories and enemas. Preferably, the oral pharmaceutical form such as tablets, capsules, dragees and granules may be a form with enteric release, such as enteric sustained release or enteric controlled release.

The pharmaceutical forms may contain at least one pharmaceutically acceptable excipient selected from fillers, binders, lubricants, glidants, disintegrants/swelling agents, solubilizers, enteric release agents, mucoadhesive components, sustained release agents, preservatives, coatings and colorants. Such excipients are known in the art of pharmaceutical formulation, and the skilled person is capable of selecting suitable excipients for the relevant pharmaceutical forms.

Suitable methods for preparing the pharmaceutical forms and compositions includes the processes of wet granulation or dry granulation of the active ingredient with the auxiliary substances and components, or direct homogenization of the active ingredient with the auxiliary substances and components.

Fillers may preferably be selected from saccharide alcohols (such as mannitol, sorbitol, xylitol), lactose, starch, pregelatinized starch, cellulose, silicified cellulose, calcium hydrogen phosphate, calcium phosphate, sucrose and calcium sulphate. The fillers may preferably be present in the amount of 5 to 90 wt. %, relative to the total weight of the composition.

Binders may preferably be selected from starch, pregelatinized starch, povidone, copovidone, hydroxypropyl methylcellulose, hydroxypropyl cellulose, ethyl cellulose, cellulose. The binders may preferably be present in the amount of 1 to 20 wt. %, relative to the total weight of the composition.

Lubricants may preferably be selected from magnesium stearate, calcium stearate, stearic acid, polyethylene glycol and sodium stearyl fumarate. The lubricants may preferably be present in the amount up to 5 wt. %, relative to the total weight of the composition.

Glidants may preferably be selected from silica, talc and sodium lauryl sulphate. The glidants may preferably be present in the amount of 0.5 to 10 wt. %, relative to the total weight of the composition.

Swelling and/or disintegrating agents may preferably be selected from crospovidone, copovidone, povidone, croscarmellose, hydroxypropyl methylcellulose, starch, pregelatinized starch, low-substituted hydroxypropyl cellulose, sodium carboxymethyl starch. The swelling / disintegrating agents may preferably be present in the amount of 1 to 50 wt. %, relative to the total weight of the composition.

Solubilizers may preferably be selected from poloxamer, sodium lauryl sulphate, polysorbate, polyoxylated oleic glycerides, glycerol monostearate and cyclodextrins. The solubilizers may preferably be present in the amount up to 30 wt. %, relative to the total weight of the composition.

Enteric release agents may preferably be selected from hydroxypropyl methylcellulose phthalate, poly(methacrylic acid-co-methyl methacrylate), cellulose acetate phthalate, poly(vinyl acetate phthalate), esters of aleuritic acid. The enteric release agents may preferably be present in the amount of 2 to 40 wt. %, relative to the total weight of the composition.

Mucoadhesive components may preferably be selected from propylene glycol alginate, sodium alginate, calcium alginate, potassium alginate, hydroxypropyl methylcellulose, sodium carmellose, polyacrylic acid, polyethylene oxide, povidone and copovidone. The mucoadhesive components may preferably be present in the amount of 5 to 70 wt. %, relative to the total weight of the composition.

Sustained release agents may preferably be selected from cellulose and cellulose ethers such as hydroxypropyl cellulose, hydroxypropyl methyl cellulose, carboxymethyl cellulose, sodium carboxymethylcellulose, ethyl cellulose, methyl cellulose, polyvinyl acetate, alginic acid, propylene glycol alginate, sodium alginate, calcium alginate, potassium alginate, polymethacrylates, guar gum, xanthan gum, carrageenan, castor oil, beeswax, carnauba wax, glycerol palmitostearate, glycerol monostearate, glycerol behenate, stearyl alcohol, polyacrylic acid. The sustained release agents may preferably be present in the amount of 5 to 70 wt. %, relative to the total weight of the composition.

The oral pharmaceutical composition may in some embodiments further contain a pharmaceutically acceptable component capable of forming carbon dioxide upon contact with gastric juices, such component may preferably be selected from carbonates and hydrogen carbonates of alkali metals and alkaline earth metals; and may preferably be present in an amount in the range from 1 to 50 wt. %, relative to the total weight of the composition.

A typical daily dose of rebamipide may range from 1 to 5000 mg for an average human (70 kg weight), more preferably from 50 to 2500 mg, even more preferably from 100 to 1000 mg, and most preferably from 300 to 500 mg. When calculating the daily dose with regard to the body weight of the person, the typical dose ranges from 15 µg/kg/day to 70 mg/kg/day, more preferably from 750 µg/kg/day to 35 mg/kg/day, even more preferably from 1.5 mg/kg/day to 15 mg/kg/day, and most preferably from 4 mg/kg/day to 7 mg/kg/day.

When immediate release formulation of rebamipide is administered, the daily dose is typically divided into several doses, which are administered separately. The daily dose may be divided into two to six separate doses taken twice daily or three times per day or four times per day or five times per day or six times per day. In a preferred embodiment the daily dose is divided into three separate doses administered three times per day, e.g. 100 mg dose administered three times per day. Alternatively, the whole daily dose can be taken at once, especially if it is in the form of a sustained release formulation, e.g. 300 mg dose administered once daily.

### Examples of carrying out the Invention

Parkinson's disease (PD) is a neurodegenerative disorder related to loss of dopaminergic neurons in *substantia nigra* (SN), basal ganglia structure in brain. Akinesia, tremor and rigidity belong to its typical, but unspecific symptoms. Although there are various diagnostic tools and approaches, including brain imaging methods or functional tests, the diagnostic proof is based on pathognomonic microanatomical changes represented basically by the *post-mortem* finding of "Lewy bodies" (LBs). LBs are formations composed of alpha-synuclein, a protein which has been suggested as one of key elements within the PD development. Accumulation and aggregation of the altered alpha-synuclein in brain is connected with further pathological changes involving mitochondrial dysfunction, oxidative stress, dysregulation of trophic factors and multiple neuro-inflammatory mechanisms. Thus, occurrence of the alpha-synuclein deposits may be accompanied by increased inflammatory markers, which was observed in previous animal studies.

Increased intestinal permeability may lead to a leakage of various substances (such as bacterial toxins and xenobiotics), that are normally unable to cross the gut wall, from the intestine into the body proper, which may trigger synthesis of misfolded alpha-synuclein in the enteric wall and its transfer to the brain via *n. vagus.* The increased intestinal permeability is a typical consequence of epithelial dysfunction caused by all sorts of enteric inflammation. Therefore, induction of the inflammatory process may be used for setting of disease models. To model such situation in animals, β-sitosterol β-D-glucoside (BSSG) model has been suggested, since consumption of dietary neurotoxins derived from the cycad seeds has been linked to the Guamanian neurological disease cluster ALS-parkinsonism dementia complex (ALS-PDC) in humans. When fed to rodents, cycad flour triggers a progressive development of neurological deficits, with behavioral and cellular features that closely approximate those observed in patients. Besides neurotoxic properties, BSSG directly interacts with epithelium. It loosens tight junctions in mucosa via increase of intracellular calcium levels and thus increases permeability of epithelium.

Male Sprague Dawley rats were used in a study performed during 10 months. The animals were put on the baseline diet consisting of BSSG, 3 mg/day, 5 doses weekly p.o. in Month 1 to Month 4. They were divided into three groups: Group A - application of rebamipide 30 mg/kg/day, applied p.o. within the standard feed 4 weeks before the BSSG exposure, and in Month 1 - Month 10; Group B - application of rebamipide 200 mg/kg/day, applied p.o. within the standard feed 4 weeks before the BSSG exposure, and in Month 1 - Month 10; and Group C - placebo, applied p.o. within the standard feed 4 weeks before the BSSG exposure; and in Month 1 - Month 4.

**Table 1: The overall study schedule**

| Procedure | Duration | Notes |
|---|---|---|
| Treatment Induction Phase | 4 weeks | Rebamipide given to groups A and B |
| Exposure/Treatment Phase | Months 0-4 (4 months) | Continual exposure by BSSG |
| | | Rebamipide treatment (group A and B) |
| | | Placebo (group C) |
| Follow-up phase | Months 4-10 | Interim functional and laboratory tests |
| | | Rebamipide treatment (group A and B) |
| Interim analysis 1 | 4^{th} month | Sacrifice of subgroup of animals, interim tests (incl. permeability test of GIT) |
| Interim analysis 2 | 7^{th} month | Sacrifice of subgroup of animals, interim tests (incl. permeability test of GIT) |
| Close-out | At month 10 | Sacrifice rest of the animals, section, final tests |

The observed parameters:
1. Quantitative analysis of misfolded α-synuclein in substantia nigra lysate
2. Quantitative analysis of misfolded α-synuclein in intestinal lysate
3. Quantitative analysis of misfolded α-synuclein in olfactory bulb lysate
4. Functional motoric tests
5. Functional behavioral tests
6. Gastrointestinal mucosa permeability tests
7. Inflammatory markers in substantia nigra (Levels of TNF-α, IL-6, etc.)
8. Inflammatory markers in serum (Levels of TNF-α, IL-6, etc.)
9. Olfactory dysfunction test
10. Retinal test
11. Mortality rate

Interim analysis 1 at Month 4 to investigate neurodegenerative disease progression: after *in vivo* olfactory dysfunction test, a subgroup of animals in each experimental group is sacrificed. *Post mortem* analysis of alpha-synuclein, histological analysis of olfactory bulb, intestine section samples, and inflammatory markers in blood samples is performed. Additionally, permeability test of gastrointestinal mucosa was performed.

Interim analysis 2 at Month 7 to investigate neurodegenerative disease progression: after *in vivo* tests (locomotion, coordination, and olfactory tests) a subgroup of animals in each experimental group is sacrificed. *Post mortem* analysis of alpha-synuclein, histological analysis of SN neurons, olfactory bulb, intestine section samples, inflammatory markers in brain section or blood samples is performed. Additionally, permeability test of gastrointestinal mucosa is performed.

Final analysis at Month 10 to investigate neurodegenerative disease progression: after *in vivo* tests (locomotion, coordination, and cognitive) a subgroup of animals in each experimental group is sacrificed. Post mortem analysis of alpha-synuclein, histological analysis of SN neurons, olfactory bulb and/or intestine is performed. In addition inflammatory markers such as TNF-α, IL-6, etc. in brain section samples and serum are measured.

Preliminary results obtained from the study indicate a significant difference between placebo and rebamipide treated groups. Placebo treated animals progressively developed motor and cognitive deficits. Olfactory dysfunction was the earliest behavioral deficit that could be observed as early as in month 4 and continued until the end of the study with no signs of recovery even after the exposure to BSSG was terminated. Motor impairments appeared about month 6 and continued to worsen over the course of the study. Histological analysis showed that alpha-synuclein aggregates appeared already at month 4 and continued to spread in various tissues in the following months.

Rebamipide treated animals showed significant improvement in parameters tested including functional tests. Interestingly, the alpha-synuclein aggregates were also reduced in comparison with the placebo group.

In order to evaluate the overall efficacy of rebamipide in Parkinson's disease a Cumulative PD Score was calculated. Following weighted parameters were considered in the analysis: quantitative analysis of misfolded α-synuclein in substantia nigra lysate, quantitative analysis of misfolded α-synuclein in intestinal lysate, quantitative analysis of misfolded α-synuclein in olfactory bulb lysate, functional motoric tests, functional behavioral tests, gastrointestinal mucosa permeability tests, inflammatory markers in substantia nigra (levels of TNF-α, IL-6, etc.), inflammatory markers in serum (levels of TNF-α, IL-6, etc.), olfactory dysfunction test, retinal test and mortality rate. Hence the Cumulative PD score represents the status of the disease and its improvement suggests efficacy.

The results of the Cumulative PD score in the rebamipide treated group were more than 20% better as compared to the placebo group, which suggests a significant improvement in the disease management. It can be concluded that rebamipide suppresses BSSG intoxication, presumably by decreasing the permeability of the gastrointestinal mucosa, and thus prevents or at least delays the onset of the symptoms and markers corresponding to Parkinson's disease as a representative of synucleinopathies.

## Claims

1. Rebamipide for use in a method of preventive treatment of a synucleinopathy in persons suffering from increased intestinal permeability or in persons who are at risk of increased intestinal permeability.

2. Rebamipide for use according to claim 1, wherein the persons suffering from increased intestinal permeability are persons suffering from at least one condition selected from low grade inflammation of the gut wall, chronic constipation or gastroparesis.

3. Rebamipide for use according to claim 1, wherein the persons at risk of increased intestinal permeability are persons suffering from stress, imbalanced diet, bacterial, viral or parasitic infection.

4. Rebamipide for use according to claim 1, wherein the persons at risk of increased intestinal permeability are persons exposed to at least one substance selected from: non-steroidal anti-inflammatory drugs, alcohol, nicotine, food additives, chemotherapeutics and antibiotics.

5. Rebamipide for use according to claim 4, wherein rebamipide is co-administered simultaneously or sequentially with non-steroidal anti-inflammatory drug, chemotherapeutic or antibiotic.

6. Rebamipide for use according to claim 4, wherein rebamipide is administered to a person abusing alcohol, nicotine or another drug.

7. Rebamipide for use according to claim 1 wherein the persons at risk of increased intestinal permeability are persons suffering from or exposed to at least one condition selected from: stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia.

8. Rebamipide for use according to any one of the preceding claims, wherein the synucleinopathy is selected from Parkinson's disease, dementia with Lewy bodies and multiple system atrophy.

9. Rebamipide for use according to any one of the preceding claims, wherein the synucleinopathy is Parkinson's disease.

10. Rebamipide for use according to any one of the preceding claims, wherein rebamipide is administered in an oral pharmaceutical form, preferably selected from tablets, capsules, dragees, granules, microgranules (sachets), orodispersible tablets or films, sublingual tablets, crushed tablets, oral solutions, oral suspensions, syrups, mouthwashes, rinses; or in a rectal pharmaceutical form, preferably selected from suppositories and enemas.

11. Rebamipide for use according to claim 10, wherein the oral pharmaceutical form is a form with enteric release, preferably enteric sustained release or enteric controlled release.

12. Rebamipide for use according to claim 10, wherein the pharmaceutical form contains rebamipide and at least one pharmaceutically acceptable excipient selected from fillers, binders, lubricants, glidants, disintegrants/swelling agents, solubilizers, enteric release agents, mucoadhesive components, sustained release agents, preservatives, coatings and colorants.

13. Rebamipide for use according to any one of the preceding claims, wherein rebamipide is administered in a daily dose of 1 to 5000 mg, more preferably from 50 to 2500 mg, even more preferably from 100 to 1000 mg, and most preferably from 300 to 500 mg.

## Patentansprüche

1. Rebamipid zur Verwendung in einem Verfahren zur vorbeugenden Behandlung einer Synucleinopathie bei Personen, die an einer erhöhten Durchlässigkeit der Darmschleimhaut leiden oder bei denen das Risiko einer erhöhten Durchlässigkeit der Darmschleimhaut besteht.

2. Rebamipid zur Verwendung nach Anspruch 1, wobei die Personen, die an erhöhter Durchlässigkeit der Darmschleimhaut leiden, Personen sind, die an mindestens einer Erkrankung leiden, ausgewählt aus einer leichten Entzündung der Darmwand, chronischer Verstopfung oder Gastroparese.

3. Rebamipid zur Verwendung gemäß Anspruch 1, wobei die Personen, bei denen das Risiko einer erhöhten Durchlässigkeit der Darmschleimhaut besteht, Personen sind, die unter Stress, unausgewogener Ernährung, bakteriellen, viralen oder parasitären Infektionen leiden.

4. Rebamipid zur Verwendung gemäß Anspruch 1, wobei die Personen, bei denen das Risiko einer erhöhten Durchlässigkeit der Darmschleimhaut besteht, Personen sind, die mindestens einer Substanz ausgesetzt sind, ausgewählt aus: nichtsteroidalen entzündungshemmenden Medikamenten, Alkohol, Nikotin, Lebensmittelzusatzstoffen, Chemotherapeutika und Antibiotika.

5. Rebamipid zur Verwendung gemäß Anspruch 4, wobei Rebamipid gleichzeitig oder nacheinander mit einem nichtsteroidalen entzündungshemmenden Arzneimittel, einem Chemotherapeutikum oder einem Antibiotikum verabreicht wird.

6. Rebamipid zur Verwendung gemäß Anspruch 4, wobei Rebamipid einer Person verabreicht wird, die Alkohol, Nikotin oder eine andere Droge missbraucht.

7. Rebamipid zur Verwendung nach Anspruch 1, wobei die Personen, bei denen das Risiko einer erhöhten Durchlässigkeit der Darmschleimhaut besteht, Personen sind, die an mindestens einer Erkrankung leiden oder dieser ausgesetzt sind, ausgewählt aus stressbedingter Gastritis, Lebensmittelvergiftung, Ungleichgewicht von Cholsäuren, HCl- und Pepsin-Sekretion im Magen, nichtinfektiösem Durchfall, Strahlentherapie, Chemotherapie, infektiöser oder postinfektiöser Beeinträchtigung der Magen-Darm-Schleimhaut, Dysmikrobie.

8. Rebamipid zur Verwendung gemäß einem der vorhergehenden Ansprüche, wobei die Synucleinopathie ausgewählt ist aus der Parkinson-Krankheit, der Demenz mit Lewy-Körperchen und der Multisystematrophie.

9. Rebamipid zur Verwendung gemäß einem der vorhergehenden Ansprüche, wobei es sich bei der Synucleinopathie um die Parkinson-Krankheit handelt.

10. Rebamipid zur Verwendung nach einem der vorhergehenden Ansprüche, wobei Rebamipid in einer oralen pharmazeutischen Form verabreicht wird, vorzugsweise ausgewählt aus Tabletten, Kapseln, Dragees, Granulat, Mikrogranulat (Beuteln), Schmelztabletten oder Filmtabletten, Sublingualtabletten, zerstoßenen Tabletten, Lösungen zum Einnehmen, Suspensionen zum Einnehmen, Sirupe, Mundwässer oder Spülungen; oder in einer rektalen Darreichungsform, vorzugsweise ausgewählt aus Zäpfchen und Einläufen.

11. Rebamipid zur Verwendung nach Anspruch 10, wobei die orale pharmazeutische Form eine Form mit enteraler Freisetzung, vorzugsweise enteraler verzögerter Freisetzung oder enteraler kontrollierter Freisetzung, ist.

12. Rebamipid zur Verwendung nach Anspruch 10, wobei die pharmazeutische Form Rebamipid und mindestens einen pharmazeutisch verträglichen Hilfsstoff enthält, ausgewählt aus Füllstoffen, Bindemitteln, Gleitmitteln, Fließregulierungsmitteln, Sprengmitteln/Quellstoffen, Lösungsvermittlern, magensaftresistenten Freisetzungsmitteln, mukoadhäsiven Komponenten, Wirkstoffen zur verzögerten Freisetzung, Konservierungsmitteln, Überzügen und Farbstoffen.

13. Rebamipid zur Verwendung nach einem der vorhergehenden Ansprüche, wobei Rebamipid in einer Tagesdosis von 1 bis 5.000 mg, bevorzugter von 50 bis 2.500 mg, noch bevorzugter von 100 bis 1.000 mg und am bevorzugtesten von 300 bis 500 mg verabreicht wird.

## Revendications

1. Rébamipide à utiliser dans une méthode de traitement préventif d'une synucléinopathie chez les personnes souffrant d'une perméabilité intestinale accrue.

2. Rébamipide à utiliser selon la revendication 1 où les personnes souffrant d'une perméabilité intestinale accrue sont des personnes souffrant d'au moins une affection choisie parmi une inflammation de faible intensité de la paroi intestinale, une constipation chronique ou une gastroparésie.

3. Rébamipide à utiliser selon la revendication 1 où les personnes à risque de développer une perméabilité intestinale sont les personnes souffrant de stress, d'une alimentation déséquilibrée, d'une infection bactérienne, virale ou parasitaire.

4. Rébamipide à utiliser selon la revendication 1 où les personnes à risque de développer une perméabilité intestinale sont les personnes exposées à au moins une substance choisie parmi : les médicaments anti-inflammatoires non stéroïdiens, l'alcool, la nicotine, les additifs alimentaires, les chimiothérapies et les antibiotiques.

5. Rébamipide à utiliser selon la revendication 4 où le rébamipide est co-administré simultanément ou séquentiellement avec un médicament anti-inflammatoire non stéroïdien, un agent chimiothérapeutique ou un antibiotique.

6. Rébamipide à utiliser selon la revendication 4 où le rébamipide est administré à une personne abusant de l'alcool, de la nicotine ou d'une autre drogue.

7. Rébamipide à utiliser selon la revendication 1 où les personnes à risque de développer une perméabilité intestinale sont les personnes souffrant de ou exposée à au moins une affection choisie parmi : la gastrite induite par le stress, l'intoxication alimentaire, le déséquilibre des acides choliques, la sécrétion gastrique de HCl et de pepsine, la diarrhée non infectieuse, la radiothérapie, la chimiothérapie, l'altération infectieuse ou post-infectieuse de la muqueuse gastro-intestinale, la dysmicrobie.

8. Rébamipide à utiliser selon l'une quelconque des revendications précédentes où la synucléinopathie est sélectionnée parmi la maladie de Parkinson, la démence à corps de Lewy et l'atrophie multisystémique.

9. Rébamipide à utiliser selon l'une quelconque des revendications précédentes où la synucléinopathie est la maladie de Parkinson.

10. Rébamipide à utiliser selon l'une quelconque des revendications précédentes où le rébamipide est administré sous une forme pharmaceutique orale, de préférence choisie parmi les comprimés, les gélules, les dragées, les granules, les microgranules (sachets), les comprimés ou films orodispersibles, les comprimés sublinguaux, les comprimés broyés, les solutions buvables, les suspensions buvables, les sirops, les bains de bouche, les bains de rinçage ; ou sous une forme pharmaceutique rectale, de préférence choisie parmi les suppositoires et les lavements.

11. Rébamipide à utiliser selon la revendication 10 où la forme pharmaceutique orale est une forme à libération entérique, de préférence à libération entérique prolongée ou à libération entérique contrôlée.

12. Rébamipide à utiliser selon la revendication 10 où la forme pharmaceutique contient du rébamipide et au moins un excipient pharmaceutiquement acceptable choisi parmi les charges, les liants, les lubrifiants, les agents de glissement, les agents désintégrants/agents gonflants, les solubilisants, les agents de libération entérique, les composants muco-adhésifs, les agents de libération prolongée, les conservateurs, les enrobages et les colorants.

13. Rébamipide à utiliser selon l'une quelconque des revendications précédentes où la dose quotidienne administrée de rébamipide est de 1 à 5000 mg, plus préférablement de 50 à 2500 mg, encore plus préférablement de 100 à 1000 mg, et le plus préférablement de 300 à 500 mg.
